# EUROPEAN PATENT APPLICATION

(11) **EP 1 955 750 A1**
(43) Date of publication of application: **13.08.2008**
(21) Application number: 08150872.3
(22) Date of filing: 31.01.2008
(51) Int. Cl.: B01D 21/01, C02F 1/56, C08B 37/00, C12H 1/02, C12H 1/04, C12C 7/14

(54) **Clarification agent**

(30) Priority: 01.02.2007 GB 0701951
(71) Applicant: Lallemand UK Limited, Burton upon Trent Staffordshire DE14 2PQ (GB)
(72) Inventor: Littlewood, John, Staffordshire Burton on Trent, Staffordshire DE13 0JR (GB)
(74) Representative: Daniels, Jeffrey Nicholas

(57) **Abstract**

A method for producing a clarification agent for potable liquors comprising:
a) providing a plant polysaccharide;
b) hydrolysing the plant polysaccharide to form a hydrolysed polysaccharide; and
c) reacting the hydrolysed polysaccharide with an amine or ammonia at an alkaline pH.

## Description

The present invention relates to a method for producing a clarification agent for potable liquors, clarification agents and uses thereof.

Collagen finings may be used in clarification or precipitation processes, for example for clarifying potable liquor such as beer, wine and cider. During the fermentation of liquor various particulate materials, such as yeast and proteins, become suspended in the liquor and need to be removed to avoid having a product which is cloudy. Collagen finings are added to the liquor to clarify it by aiding the precipitation of the suspended materials. A filtration step generally follows (the main exception being traditional cask beers where sediment separates and remains in the cask). It is important to achieve effective precipitation to enable good separation of the clarified liquor from the precipitate. For example, in the case of beer, collagen finings can be added as a solution to the fermented beer in cask, and the beer is allowed to clarify before being dispensed. Similarly, the finings are used to clarify bulk beers prior to processing.

Collagen finings are generally prepared from fish isinglass, which constitutes a very pure source of collagen prepared from the swim bladders of fish. Isinglass is widely used in the brewing industry, sometimes in conjunction with auxiliary fining agents. Isinglass is believed to carry both positive and negative charges at beer pH which enables it to interact with negatively charged yeast cells plus beer proteins which carry positive charges, inducing flocculation and sedimentation of the resultant larger particles, leaving a clearer supernatant. Clarification with isinglass generates relatively compact sediment, which is advantageous since it means that less beer or wine is wasted when it is decanted.

The removal of solids by isinglass enables cost-savings for example with regard to beer filtration, because the filter can be operated for longer before it blinds which reduces the frequency of stopping to reset the filter bed which entails time and increased consumables costs (e.g. filter cards and filter powders).

Auxiliary finings may also be used to accelerate the action of isinglass and cause more rapid coagulation. The use of auxiliary finings comprising an aqueous extract of carrageenan moss, or an alkali metal salt of its active principle, namely alginic acid, are known in the prior art (GB 912492).

Isinglass is the established beer clarifier, and is widely used, but it has some disadvantages. In particular, it is of animal (fish) origin. This makes potable liquor prepared using isinglass unattractive to certain consumer groups. As a result, there is a need for the development of a new type of clarification agent which is vegetable-based and non-allergenic.

Various studies by other groups looking for alternatives to isinglass have focused on the use of pectin as an alternative clarification or fining agent. The African Sisal & Produce Co. Ltd filed a provisional specification for a UK patent in 26^{th} January 1942 in which claims were made that "polyuronides" (examples quoted as pectin and algin) - preferably as partially satisfied soluble salts (sodium/potassium/ammonium) were as effective as isinglass in beer clarifying. Examples describe the manufacture of a clarification agent by the partial hydrolysis of pectin using NaOH followed by neutralisation. This clarification agent is indicated to be sufficient to clarify beer. Further, it is stated in the application that the alkaline salts of alginic acid give similar results.

Further work with pectin has been completed by Laporte BSD Limited. WO 98/00516 describes a composition suitable for use as a fining agent characterised in that it comprises pectin in a molecular size range indicated by limiting viscosity number less than 0.5 and preferably from 0.005 to 0.2. This pectin may be derived from apple, citrus, beet, carrot, potato or sunflower and converted to the required form by modification with pectinase under conditions suitable to attain the required limiting viscosity number. The application discloses that the composition is particularly suitable for fining beers or lagers. However, although this work was published in 1998, as far as the present applicant is aware, pectin is still not commonly used as a clarification agent in the brewing industry. In particular, the present applicants have not found it to be as effective as isinglass.

WO 2006/032088 also discloses finings formulations comprising pectin and methods of using pectin in a finings process to produce fined beverages, particularly beers. In particular, the finings formulations disclosed comprise a pectin and a donor of sulphur dioxide.

However, further clarification agents to isinglass are still being sought.

Accordingly, in a first aspect there is provided a method for producing a clarification agent for potable liquors comprising:
a) providing a plant polysaccharide;
b) hydrolysing the plant polysaccharide to form a hydrolysed polysaccharide; and
c) reacting the hydrolysed polysaccharide with an amine or ammonia at an alkaline pH.

The invention further provides a clarification agent for potable liquors comprising a hydrolysed plant polysaccharide which has been reacted with an amine or ammonia at an alkaline pH, a method of clarifying a potable liquor comprising the step of contacting the clarification agent of the invention with liquor, and use of the clarification agent of the present invention for the clarification of potable liquors.

It has been surprisingly found by the present applicants that a clarification agent comprising a hydrolysed plant polysaccharide which has been reacted with an amine or ammonia at an alkaline pH may be used for the clarification of potable liquors. The clarification agent of the present invention is particularly advantageous since it avoids the need for the use of animal derived products. Moreover, potable liquors treated with the clarification agents of the present invention surprisingly have a better filterability in comparison even to current isinglass products. This is advantageous because the better the filterability, the longer the filter run. This means that filters used in the processing of the potable liquors need to be changed less frequently, resulting in lower processing costs.

In a preferred aspect of the present invention, the term "clarification" as it is used herein refers to a particle reducing process which acts by increasing said particle size and thus increasing the rate of gravity driven sedimentation. In particular, the clarification agent according to the present invention is soluble. As such it can be dispersed within the liquid to be clarified in order to initiate aggregation of the solid particles within the liquid. This allows it to be used in a pre-filter treatment or as a haze reducing step.

The plant polysaccharide may be a structural plant polysaccharide or a gum. Plant polysaccharides according to the present invention include pectinate (pectin), alginate (algin), carrageenan, cellulose, hemicellulose and carboxymethylcellulose, although in a preferred aspect of the invention the plant polysaccharide is not pectin. In a particularly preferred aspect of the present invention the polysaccharide comprises an alginic acid or an alginate salt, more preferably sodium alginate.

An alginate, or algin, is a salt of alginic acid: a linear polymer consisting of ( 1→ 4)-β-linked D-mannuronic acid residues and (1→ 4)-α-linked L-guluronic acid residues. Alginates occur in the cell wall and intercellular mucilage in marine brown algae (*Phaeophyceae*), and are block co-polymers of widely varying composition and sequence. Alginate is widely used industrially because of its ability to retain water, and its gelling, viscosifying and stabilising properties (Draget, K.I. Alginates. Handbook of hydrocolloids. 379 - 395 2000). As such it is commercially manufactured and can easily be obtained (for example, some commercial sources for alginates are Snap Natural and Alginate Products Ltd, FMC Biopolymer, Danisco).

Step b) of the method according to the present invention includes both partial and full hydrolysis of the plant polysaccharide. In one aspect, this step may comprise chemical hydrolysis, using, for example, acid or alkaline pH. It is preferred to use a high pH. This may be achieved by the use of a base. A particularly preferred base is ammonia.

In an alternative embodiment, step b) in the method comprises enzymatic hydrolysis. In a preferred aspect of the invention, where the plant polysaccharide is an alginate salt, the hydrolysis is effected using the enzyme alginate lyase (alginase); where the plant polysaccharide is pectinate (pectin) the enzyme is a pectinase; where the polysaccharide is carrageenan the enzyme is a carrageenase; where the polysaccharide is a cellulose, hemicellulose or carboxymethylcellulose the enzyme is a cellulase.

In step c) of the method of the present invention the hydrolysed polysaccharide is reacted with an amine or ammonia at an alkaline pH. Amines are derivatives of ammonia in which one or more hydrogen atoms have been replaced with hydrocarbon groups. The role of the amine or ammonia used in this step is to provide a source of amino nitrogen and to introduce nitrogen into the polysaccharide. The amines are typically primary amines. In a preferred aspect of the present invention the hydrolysed polysaccharide is reacted with an amino acid, more preferably a naturally-occurring amino acid such as glycine. In particular, the reaction is conducted at pH 10 or over.

The alkaline pH can be created in a number of ways that are well known in the art. Where ammonia is used as the source of amino nitrogen the alkaline pH is created without the addition of further chemicals. In particular, the ammonia is capable of raising the pH above pH 10. Alternatively, the pH of the mixture can be raised by the addition of further chemicals such as alkaline buffers. For example, where glycine is used to provide the amino nitrogen, sodium hydroxide can also be added to create an alkaline pH.

As is clear to the skilled person from the above paragraphs, steps b) and c) in the method are conducted in a hydrous environment.

Although the present inventors do not wish to be bound by theory, it is proposed that in step c) amide bonds are formed between amines and carboxyl groups in the hydrolysed plant polysaccharide, such as in protein synthesis. It is proposed that the alkaline pH assists the degradation and the destabilisation of the hydrolysed polysaccharide and is instrumental in facilitating the reaction with the amino nitrogen.

In particular, the method of producing a clarification agent according to the present invention produces a soluble biopolymer-based agent.

In one aspect of the present invention the reaction is amidation. In particular, the method of producing a clarification agent according to the present invention comprises alkaline hydrous amidation.

In one embodiment of the method for producing a clarification agent for potable liquors according to the present invention step b) and step c) are performed together with ammonia. In a preferred aspect of this embodiment the plant polysaccharide provided in step a) is alginic acid which is insoluble in water. Step b) and step c) are performed by adding ammonia. The ammonia dissolves the alginic acid and creates a solution the pH of which is above 10. It is considered that alginic acid is negatively charged due to its carboxyl groups and readily interacts with the positively charged ammonium ions. As demonstrated in the Examples section, the product produced by this method shows good clarification ability in beer.

In an alternative embodiment of the method for producing a clarification agent for potable liquors according to the present invention step b) and step c) are performed as separate steps. In a preferred aspect of this embodiment the plant polysaccharide provided in step a) is an alginic salt and step b) is performed by dissolving the alginic salt in water and adding alginate lyase. The action of this enzyme to the alginate salt produces products which include α-L-guluronate and β-D-mannuronate by cleaving the beta-1,4 glycosidic linkages between the units of the polymer to produce oligomers. In particular, the enzyme is known to cleave at the beta-(1-4)-D-mannuronic bonds residues to yield oligosaccharides with 4-deoxy-a-L-erythro-hex-4-enopyranuronosyl groups at their non-reducing terminus. Step c) is performed either by adding ammonia (which also raises the pH to above 10), or another source of amino nitrogen such as glycine (plus sodium hydroxide to raise the pH above 10). It is considered that the ammonia/glycine will presumably be interacting with negatively charged groups that become exposed due to the action of the enzyme plus the further degrading action of the alkaline pH. The inventors do not wish to be bound by theory but it is considered that amidation could be occurring between the amino nitrogen provided by the ammonia or primary amine and carboxyl groups on the alginate.

The alkaline pH is sufficiently high to produce a product which is an effective clarification agent. For the purpose of the present application, an effective clarification agent is one which in a clarification test gives a result comparable or equivalent to that produced with isinglass, the benchmark clarifier. Such tests are well known to a person skilled in the art; isinglass is included in comparisons at its typical use rates, along with an untreated/blank beer sample to show that the isinglass is capable of clarifying the beer. The trial products are then added over a range of rates to see if they can produce any clarification and if so how close is it to isinglass' performance in terms of clarity/EBC haze, sediment compaction and filterability. Such a test is set out in Example 1.

In a preferred aspect of the invention the alkaline pH is at least 10. This pH provides a more successful reaction with the amine or ammonia, especially for hydrolysed alginates.

The method according to the present invention may further comprise the steps of precipitating the hydrolysed polysaccharide which has been reacted with the amine or ammonia, drying the precipitate and producing a powdered version of the clarification agent. Such a dried product may be easier to transport and store, and may be reconstituted as a liquid at point of use.

The clarification agent of the present invention can be used to clarify liquor. In particular, the liquor may be selected from beer, ale, lager, cider, wine or a derivative of beer, ale, lager, cider or wine.

The present invention will be described further by way of example only, with reference to the following Figures:
Figure 1 is a graph showing the filterability of post-centrifuge lager 1 following clarification with a clarification agent of the present invention (at 1.25 % v/v addition rate of the clarification agent to the beer) in comparison to that of post-centrifuge lager 1 clarified with isinglass (at 0.4 % v/v addition rate of the isinglass to the beer).The clarification agent according to the present invention shown is sodium alginate treated with alginate lyase and then reacted with ammonia. The table below the graph shows the haze reading in EBC at zero degrees Celsius.
Figure 2 is a graph showing the filterability of post-centrifuge lager 2 following clarification with a clarification agent of the present invention in comparison to that of lager 2 clarified with CBF211 (a ready-for-use liquid isinglass finings product produced by AB Vickers).
Figure 3 is a graph showing the filterability of post-centrifuge lager 3 following clarification with a clarification agent of the present invention in comparison to that of lager 3 clarified with ready-for-use (RFU) isinglass.

### EXAMPLES

The performance of clarification agents in the following experiments is assessed in relation to the following criteria:
a) ability to clarify beer (speed plus visual and turbidimeter readings and nature of sediment produced)
b) effect on filterability of beer using small scale filter rig
c) ability to clarify wort (visual and turbidimeter readings)

### Example 1 - Preparation of clarification agent and comparison with other potential clarification agents

Samples of commercially available alginic acid (from Quingdao Jiaonan Bright Moon Seaweed Industrial Company Ltd), sodium alginate (from FMC Biopolymer) and ammonium alginate (from Snap Natural and Alginate Products Ltd) were assessed for beer clarification activity but were found not to be effective (see Tables below). Each was then treated with alginate lyase, (from Sigma-Aldrich) which still did not enable clarification. Then the effects of using alginate lyase plus ammonia were assessed.

In particular, a sample was prepared by dissolving sodium alginate in hot water (the temperature of the water is not believed to be critical but is in the region of 80 to 90 °C) to create a 2 % w/v solution; the solution was cooled to 40°C; 3ml of a 0.04g/l alginate lyase dilution was added to 100ml of the 2 % w/v sodium alginate solution; the solution was mixed for 30 minutes (the viscosity visibly decreases over this time); 200 ml of 10% ammonia solution was added to 100 ml of enzyme-treated 2 % w/v solution which raised the solution to pH 10. The resultant solution contained approx. 0.65 % w/v sodium alginate and 2 % w/v ammonia.

As an alternative to the method described in the above paragraph, glycine dissolved in water can be used in the place of the ammonia solution. 100 ml of enzyme-treated 2 % w/v sodium alginate can be mixed with 200ml 10% w/v glycine, and then 12.5ml of 40% w/v sodium hydroxide solution added to get the pH above 10. The glycine in the final product would be approximately 6.4% w/v, although it is probable that some of this forms links with the alginate oligomers.

The sample clarification agents were tested on post-centrifuge lager 1, post-centrifuge lager 2, cask ale from racking tank prior to clarifiers being added, and cask ale wort prior to clarifiers being added.

The post-centrifuge lagers 1, 2 & 3, are different samples of lager-style beers produced commercially by major international brewing companies. The post-centrifuge aspect means that they have been centrifuged in the brewery as per standard practice - centrifuging removes the yeast from the beer to improve its filterability. Since the beer proteins are not removed by the centrifuging, isinglass is frequently added to such beers (including the 3 types used here) after centrifuging and allowed to react for a period of time (typically from one day up to around a week) in order to remove much of this protein and improve the filterability of the beer, bringing the cost-savings mentioned earlier. In the present examples, however, treating these post-centrifuge lagers with isinglass is compared with treating the lagers with the current invention. The results show that the clarification agents of the present invention have the potential to yield a beer/lager which is even more readily-filtered than beer/lager which has been isinglass-fined.

As is known by a person skilled in the art "racking tank" is a commonly-used conditioning or intermediate storage vessel in the brewery, into which cask ale is transferred after fermentation - the process of separating the liquid beer from the sediment by pumping to another vessel is known as "racking". The beer is racked off into the racking tank. Typically, a brewery will then add clarifiers - auxiliary finings followed by isinglass finings - either into the racking tank or en route to it. Also some prefer to make the isinglass additions after this tank or even into the individual casks. "Wort" is the name of an earlier intermediate in the brewing process - it is the term for the liquid that results after the boiling step in a vessel called a kettle, which occurs prior to the fermentation step. Typically the wort has an addition of carrageenan approximately 10 minutes prior to the end of the boil which reacts with and flocculates proteins - i.e. it clarifies the wort which leads to fewer turbidity problems further down the line but does not replace the use the beer clarifiers (i.e. auxiliary and isinglass finings) - it is additional to these.

The table below shows the results for post-centrifuge lager 1 in which rate % is the % v/v clarifier added to the beer i.e. 0.35 % is 0.35ml/100ml. Clarity was measured using a Hach turbidimeter calibrated by the Institute of Brewing recommended method. Comments on visual appearance of sediment were made with "firm" and "compact" being the desired outcomes.

| **Addition** | **Rate %** | **Haze EBC** | **Sediment** |
|---|---|---|---|
| blank | | 12.4 | |
| RFU isinglass | 0.35 | 3.82 | firm/compact |
| sodium alginate | 0.5 | 17.8 | none |
| sodium alginate | 1 | 20.2 | surface gel |
| sodium alginate | 2 | 22.5 | surface gel |
| sodium alginate + ammonia | 0.5 | 9.1 | none |
| sodium alginate + ammonia | 1 | 7.35 | loose/surface gel |
| sodium alginate + ammonia | 2 | 6.1 | ~ 50% of beer volume |
| sodium alginate + alginate lyase | 0.5 | 15.6 | none |
| sodium alginate + alginate lyase | 1 | 16.4 | none |
| sodium alginate + alginate lyase | 2 | 21 | none |
| sodium alginate + alginate lyase + ammonia | 0.5 | 15.3 | none |
| sodium alginate + alginate lyase + ammonia | 1 | 3.8 | compact/firm ~ 5% of beer volume |
| sodium alginate + alginate lyase + ammonia | 2 | | ~ 80% beer volume (top 20% visually bright) |
| alginic acid + ammonia | 0.5 | 41.4 | none |
| alginic acid + ammonia | 1 | 36 | negligible |
| alginic acid + ammonia | 2 | 3.8 | ~ 10% beer volume, loose |

This example shows that sodium alginate is not effective alone but when treated with ammonia it has a clarification effect but produces very heavy sediments. Also shows that sodium alginate plus alginase and ammonia is a good clarifier and the sediment is much more compact (at around a 1 % v/v addition rate) than sodium alginate plus ammonia or alginic acid plus ammonia (the latter also clarifes).

Table showing results on cask ale from racking tank (cask beer):

| **Addition** | **Rate %** | **Initial settlement haze EBC** | **Resettled haze EBC** | **Sediment** |
|---|---|---|---|---|
| blank | | 6.8 | 7.1 | |
| RFU isinglass | 1.2 | 0.9 | 1.21 | firm/compact |
| sodium alginate | 0.5 | 9.35 | 8 | woolly precipitate across depth of bottle |
| sodium alginate | 1 | 10.7 | 8.14 | woolly precipitate across depth of bottle |
| sodium alginate | 2 | 9.77 | 7.07 | woolly precipitate across depth of bottle |
| sodium alginate + ammonia | 0.5 | 5.72 | 3.78 | woolly precipitate across depth of bottle |
| sodium alginate + ammonia | 1 | 3.41 | 1.89 | woolly precipitate across depth of bottle |
| sodium alginate + ammonia | 2 | 4.35 | 2.68 | very heavy/loose |
| sodium alginate + alginate lyase | 0.5 | 8.62 | 7.74 | None |
| sodium alginate + alginate lyase | 1 | 9.55 | 8.35 | None |
| sodium alginate + alginate lyase | 2 | 11.6 | 9.45 | None |
| sodium alginate + alginate lyase + ammonia | 0.5 | 2.06 | 2.02 | slightly heavy/loose |
| sodium alginate + alginate lyase + ammonia | 1 | 1.92 | 1.9 | slightly heavy/loose |
| sodium alginate + alginate lyase + ammonia | 2 | 2.21 | 2.22 | slightly heavy/loose |
| alginic acid + ammonia | 0.5 | 2 | 2.48 | firm/compact |
| alginic acid + ammonia | 1 | 2.61 | 2.33 | slightly heavy/loose |
| alginic acid + ammonia | 2 | 10.1 | 3.16 | none |

In the cask beer, again the sodium alginate treated with alginase plus ammonia shows promise as a clarifier, although it does not produce quite the same clarity as the current rate of isinglass (plus sediments are less compact).

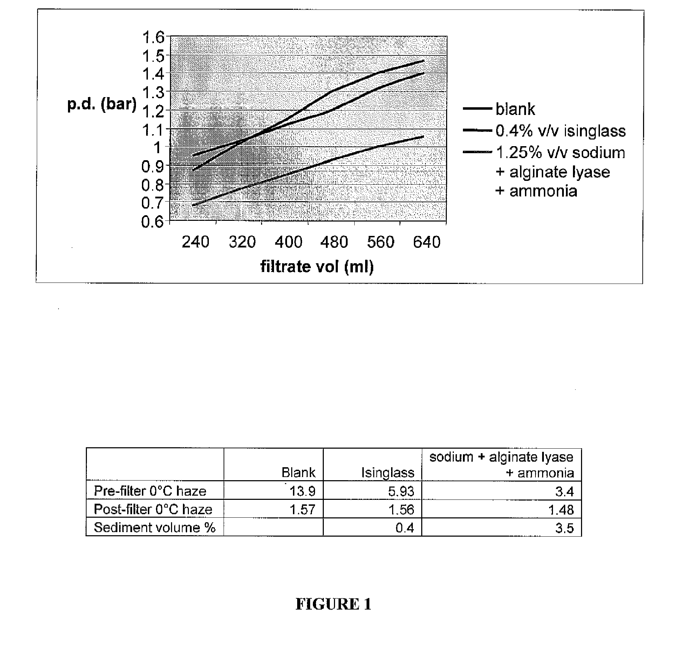

The filterability in post-centrifuge lager 1 is shown in Figure 1. The treated alginate showed good filterability improvement in this beer and good clarification but relatively heavy sediment. The graph shown in Figure 1 indicates the pressure differential (p.d.) across the filter during filtration (with the pressure measured in bar). The faster the pressure differential increases through the filtration, the quicker the filter blinds/blocks. Accordingly, better filterability is achieved if the pressure differential increases gently or remains low for as long as possible to enable more beer to be filtered before the filter blocks and has to be stripped, cleaned and re-set.

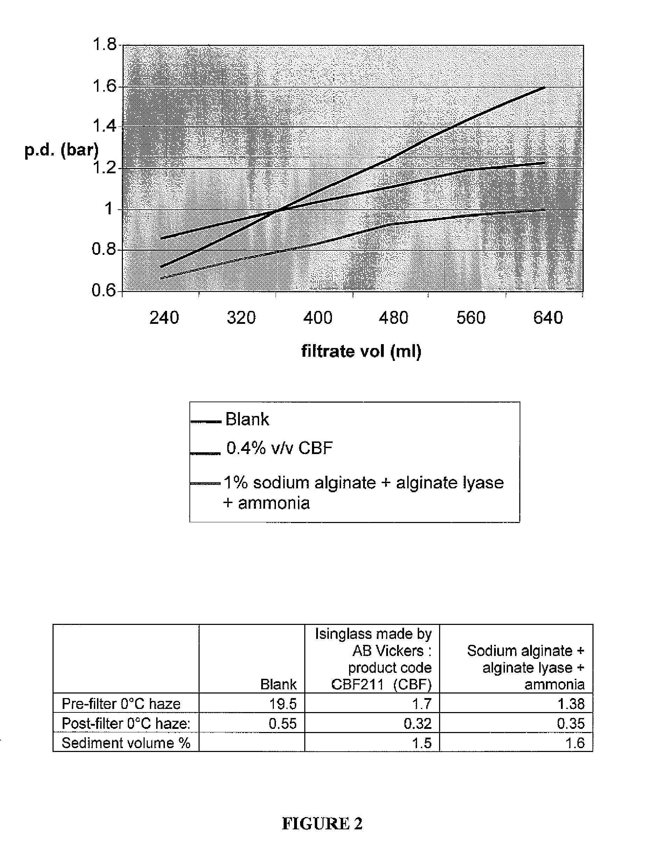

The filterability in post-centrifuge lager 2 is shown in Figure 2. The treated alginate showed good filterability improvement in this beer and good clarification plus similar sediment to isinglass.

### Effect in cask ale wort:

The table below shows that the treated alginate clarifies wort effectively and it also produced similar sediment volumes to the Compac (carrageenan) Tablets (CGT311) Compac Tablets, code CGT311, is an AB Vickers kettle finings product which brewers add to the wort-boiling stage to help to clarify the wort by precipitation of protein, as referred to earlier.

| **Addition** | **Rate/%** | **Haze EBC** |
|---|---|---|
| blank | 0 | 45.3 |
| Compac Tablet | 5ppm | 42.3 |
| Compac Tablet | 10ppm | 28.4 |
| Compac Tablet | 15ppm | 28.2 |
| Compac Tablet | 20ppm | 21.3 |
| Compac Tablet | 25ppm | 11.3 |
| Compac Tablet | 30ppm | 8.7 |
| sodium alginate + alginate lyase + ammonia | 0.50% | 29.5 |
| sodium alginate + alginate lyase + ammonia | 1% | 13.5 |
| sodium alginate + alginate lyase + ammonia | 2% | 8.35 |

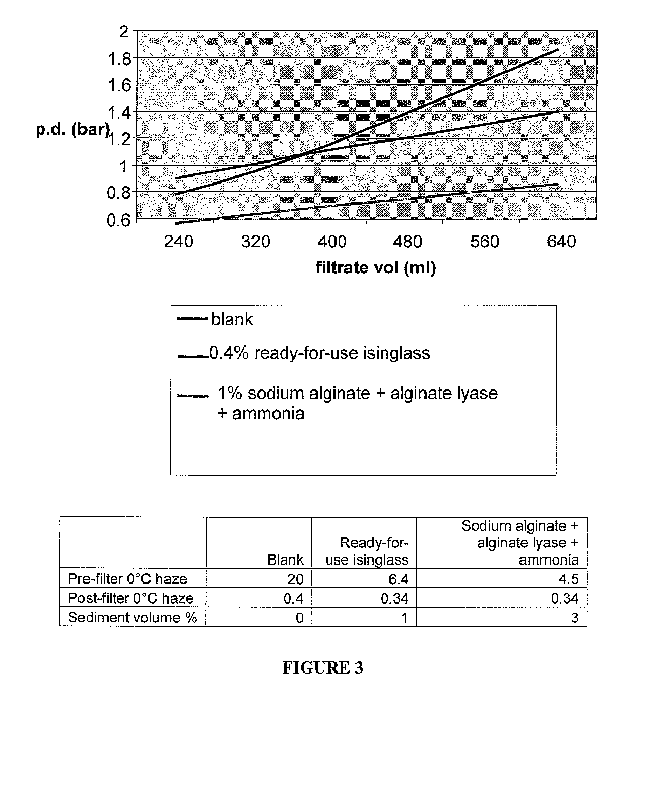

The filterability in post-centrifuge lager 3 is shown in Figure 3. The treated alginate again showed good filterability potential and clarification but heavier sediment.

In summary, the clarification product of the present invention has good potential in the clarification and filterability of beers. It has the advantages of not being derived from animal products and it can be based on food-grade raw material. Further, it has been shown to clarify wort as well. In addition, the present inventors have surprisingly found that the filterability is even better than the current isinglass products. This allows for longer filter runs, leading to cost and time savings.

## Claims

1. A method for producing a clarification agent for potable liquors comprising:
a) providing a plant polysaccharide;
b) hydrolysing the plant polysaccharide to form a hydrolysed polysaccharide; and
c) reacting the hydrolysed polysaccharide with an amine or ammonia at an alkaline pH.

2. A method according to claim 1 wherein the polysaccharide is partially hydrolysed.

3. A method according to claim 1 or claim 2 wherein the step of hydrolysing the polysaccharide is effected using an enzyme.

4. A method according to any preceding claim wherein the plant polysaccharide is an alginate salt or alginic acid.

5. A method according to claim 4 wherein the alginate salt is sodium alginate.

6. A method according to claim 4 or claim 5 wherein the step of hydrolysing the polysaccharide is effected using alginate lyase.

7. A method according to any preceding claim wherein the alkaline pH is at least 10.

8. A method according to any preceding claim further comprising the steps of precipitating the hydrolysed polysaccharide which has been reacted with an amine or ammonia, drying the precipitate and producing a powdered form of the clarification agent.

9. A clarification agent produced by the method of any one of claims 1 to 8.

10. A clarification agent for potable liquors comprising a hydrolysed plant polysaccharide, which has been reacted with an amine or ammonia.

11. A clarification agent according to claim 10 wherein the plant polysaccharide is an alginate.

12. A clarification agent according to claim 10 or claim 11 wherein the plant polysaccharide is reacted with glycine.

13. A method of clarifying a potable liquor comprising the step of contacting the clarification agent of any one of claims 9 to 12 with the liquor.

14. A method according to claim 13 wherein the liquor is beer, ale, lager, cider, wine or a derivative of beer, ale, lager, cider or wine.

15. Use of the clarification agent of any one of claims 9 to 12 for the clarification of potable liquor.
